# FASCICULE DE BREVET EUROPEEN

(11) **EP 2 237 734 B1**
(45) Date de publication et mention de la délivrance du brevet: **29.06.2016**
(21) Numéro de dépôt: 09711266.8
(22) Date de dépôt: 09.02.2009
(51) Int. Cl.: A61F 13/00, A61M 1/00, A61B 17/132

(54) **DISPOSITIF DE CONTROLE HEMOSTATIQUE D'UN ECOULEMENT SANGUIN**
VORRICHTUNG ZUR HÄMOSTATISCHEN BLUTFLUSSKONTROLLE
DEVICE FOR HAEMOSTATIC CONTROL OF A BLOOD FLOW

(30) Priorité: 08.02.2008 FR 0800676
(43) Date de publication de la demande: 13.10.2010
(73) Titulaire: Université Joseph Fourier, 38041 Grenoble Cedex 09 (FR)
(72) Inventeur: BLIN, Dominique, F-38700 La Tronche (FR)
(74) Mandataire: Grosset-Fournier, Chantal Catherine
(86) Numéro de dépôt international: PCT/FR2009/050198
(87) Numéro de publication internationale: WO 2009/101348

(56) Documents cités:
- WO-A-2006/048246
- WO-A-2006/105892
- WO-A-2007/133644
- WO-A-2008/141228
- WO-A1-2007/068477
- US-A- 5 163 944
- US-A- 5 224 947
- US-A1- 2008 306 456
- US-B2- 7 216 651

## Description

La présente invention est relative à un dispositif de contrôle hémostatique d'un écoulement sanguin, susceptible de se produire, tant au cours d'une opération chirurgicale sur un organe délicat, notamment sur le coeur, qu'en cas de lésion vasculaire accidentelle.

Lors d'une opération chirurgicale, plus encore en cas de blessure accidentelle grave, l'hémostase de saignements importants pose généralement quatre types de problèmes.

En premier lieu, la plaie peut être mal visualisable en cas de saignement notable ; elle peut aussi être difficile à suturer du fait de la fragilité des tissus lésés.

De plus, il n'est pas normalement possible de contrôler la plaie par clampage ; enfin, elle peut être située dans une zone dangereuse qui nécessite une compétence chirurgicale toute particulière.

Ainsi, ces hémorragies peuvent être de nature à perturber le champ opératoire si elles ne sont pas contrôlées et limitées, voire même totalement arrêtées, exigeant donc une hémostase immédiate par une intervention usuelle de compression ou d'électrocoagulation, ou encore par une opération consistant à pincer les deux extrémités de la plaie pour l'aider à cicatriser plus rapidement, ces interventions pouvant être effectuées cumulativement en suivant ou séparément selon les circonstances.

Or, dans un cas comme dans l'autre, l'hémorragie peut néanmoins subsister pendant une durée plus ou moins importante et avec un écoulement du sang plus ou moins grand.

De plus,comme déjà mentionné plus haut, dans certaines éventualités, le saignement intervient en zone inaccessible ou difficilement accessible pour le chirurgien, ce qui complique la vision de la plaie, ou encore dans des endroits où les tissus sont très fragiles, rendant leur réparation peu aisée.

Pour pallier ces inconvénients et permettre dans les meilleures conditions possibles une hémostase du tissu lésé siège d'une hémorragie, on a déjà prévu de mettre en oeuvre une méthode qui se révèle souvent satisfaisante, surtout si la blessure est relativement superficielle.

On a notamment déjà envisagé, pour traiter des plaies superficielles de ce genre, infectées ou non, une méthode qui consiste à réaliser au droit de la plaie ou contre le tissu mis à nu, un drainage aspiratif qui provoque une évacuation des particules mortes infectées et une certaine migration des tissus favorisant l'hémostase, voire, en cas de blessure légère, la guérison possible de la plaie.

Toutefois, les réalisations proposées, dont les brevets WO 2006/048 246, US 5 645 081, 7 198 046, 7 216 651 ou 2004/0243073 illustrent diverses variantes, sont surtout adaptées pour des plaies relativement superficielles, avec des lésions tissulaires moins graves, grâce à la mise en oeuvre d'une cloche ou d'un pansement étanche appliqué sur le pourtour de la zone blessée pour créer un espace clos au-dessus de celle-ci, relié à une source de vide extérieure, le cas échéant via des moyens de soutirage des exsudats ou de filtrage des impuretés, pendant une durée notable. Les préambules des revendications 1 et 8 sont basés sur les dispositifs connus des documents WO 2007/068477 et US 72/665/ respectivement.

La présente invention vise à perfectionner la technique connue de l'aspiration sous vide d'une zone d'un tissu, siège d'une hémorragie, notamment dans le cas où l'écoulement sanguin est important et se produit sur un organe interne fragile, au moyen d'un dispositif qui permet de réaliser et de contrôler cette aspiration afin d'obtenir une hémostase efficace, rapide et sûre, apte à être maintenue en l'état aussi longtemps que nécessaire pour permettre d'achever l'intervention chirurgicale entreprise ou le traitement de la blessure soignée, dans des conditions d'intervention et notamment de vision de la plaie qui soient optimales, en éliminant pour l'essentiel l'écoulement sanguin qui risque de la perturber.

A cet effet, le dispositif considéré comporte une ventouse de recouvrement étanche, apte à entourer et à s'appliquer étroitement contre une zone de tissu à traiter siège d'une hémorragie ou d'un écoulement sanguin à contrôler, cette ventouse venant en contact avec la périphérie de la zone de tissu et délimitant un volume interne relié de façon étanche par un cathéter ou un tube de liaison similaire à un organe extérieur d'aspiration et de mise sous vide, créant dans ce volume, entre la ventouse et la zone de tissu, une dépression de valeur déterminée, la ventouse présentant une formé générale de coupelle, dont le fond supporte au moins un organe d'appui interne, apte à venir se disposer en regard de la zone de tissu sous la ventouse.

Grâce à ces dispositions, en elles mêmes connues, la plaie se referme par migration des tissus sous l'effet de la dépression créée, l'organe d'appui interne maintenant le tissu sensiblement en place à la base de la coupelle.

Selon l'invention et dans une première forme de réalisation, l'organe d'appui interne est constitué par un obturateur plein, solidarisé du fond de la ventouse.

Dans un mode d'exécution préféré, la coupelle de la ventouse présente un profil hémisphérique autour d'un axe de révolution selon la direction duquel s'étend l'obturateur, disposé sensiblement au centre de la coupelle.

De préférence également, l'obturateur est en forme de cylindre droit dont l'axe est confondu avec l'axe de révolution de la coupelle.

L'obturateur présente une hauteur qui est sensiblement supérieure à celle de la coupelle à profil hémisphérique, de manière à exercer une compression sur la zone de tissu lorsque le bord de cette coupelle, opposé à son fond, est appliqué sur cette zone. Le cas échéant, la hauteur de l'obturateur peut être légèrement inférieure à celle de la coupelle, pour faciliter le positionnement de celle-ci contre la zone de tissu.

L'obturateur comporte, dans le fond de la coupelle, un organe de raccordement étanche avec le tube de liaison relié à l'organe extérieur de mise sous vide et au moins un orifice de communication avec le volume interne de la coupelle pour l'aspiration de l'air contenu dans ce volume.

Selon le cas, la coupelle et l'obturateur peuvent être simultanément venus de fabrication pour former un ensemble monobloc; éventuellement, la coupelle et l'obturateur sont réalisés séparément puis collés mutuellement dans le fond de la coupelle.

Par ailleurs et en règle générale, le diamètre de l'obturateur est sensiblement égal à la moitié du diamètre de la coupelle au droit de son bord appliqué contre la zone de tissu.

Dans une autre forme de réalisation de l'invention, l'organe d'appui interne est constitué par une grille perforée présentant une pluralité de trous de passage permettant la libre traversée de l'air dans le volume de la coupelle avant son évacuation dans le tube de liaison par l'organe extérieur de mise sous vide.

De préférence, les trous de passage de la grille sont identiques ou non, circulaires ou présentent un autre profil et le cas échéant sont régulièrement répartis dans son étendue.

Le cas échéant, la périphérie de la grille peut comporter des dents d'impactage en relief, de faible hauteur, propres à pénétrer légèrement dans la couche superficielle de la zone de tissu, lors de l'application de la coupelle sur celle-ci.

Selon une caractéristique également avantageuse et quel que soit le mode de réalisation choisi, le bord de la coupelle opposé à son fond, comporte une collerette d'appui plane, circulaire, en contact avec la zone de tissu qu'elle entoure. Avantageusement, cette collerette présente un profil sensiblement conique sur l'axe de la coupelle.

La coupelle peut être souple et réalisée en un matériau du genre résine silicone ou analogue, biocompatible avec la zone de tissu à traiter. Toutefois, elle peut être constituée au moyen d'un matériau rigide, du type résine epoxy durcie, avec un revêtement de protection adhésif en silicone.

En fonction de l'importance de la zone de tissu à traiter et notamment du type d'hémorragie ou de l'écoulement sanguin à contrôler, le diamètre de la coupelle au droit de son bord appliqué contre cette zone peut être variable d'une réalisation à l'autre et notamment être compris entre 5 et 60 mm, de préférence et en moyenne de l'ordre de 50mm. De même et selon le cas, la hauteur de la coupelle peut varier entre 10 et 30mm.

Selon encore une autre caractéristique, l'organe extérieur de mise sous vide est constitué par une pompe ou un bocal d'aspiration du type « Redon », la dépression créée pouvant être variable dans des limites notables mais étant usuellement comprise entre 500 et 700 mm de Hg.

D'autres caractéristiques d'un dispositif de contrôle hémostatique conforme à l'nvention, apparaîtront encore à travers la description qui suit de plusieurs exemples de réalisation, donnés à titre indicatif et non limitatif, en référence aux dessins annexés sur lesquels :
- La Figure 1 est une vue schématique en perspective d'un organe vivant, -ici un coeur, présentant une zone de tissu siège d'un hémorragie localisée, apte à être contrôlée au moyen d'une ventouse conforme à l'invention.
- La Figure 2 est une perspective à plus grande échelle, illustrant la ventouse considérée, vue de dessus, selon un premier mode de réalisation de l'invention où l'organe d'appui interne est constitué par un obturateur plein.
- La Figure 3 représente la ventouse de la Figure 2, vue de dessous.
- La Figure 4 est une vue éclatée, en élévation, d'une variante de la ventouse des Figures 2 et 3, où l'organe d'appui interne est constitué par une grille perforée.
- La Figure 5 illustre une modification de la grille représentée sur la Figure 4.
- Les Figures 6 et 7 sont des schémas de principe, illustrant le fonctionnement de la ventouse pour réaliser l'hémostase de la zone de tissu sur laquelle elle est appliquée.

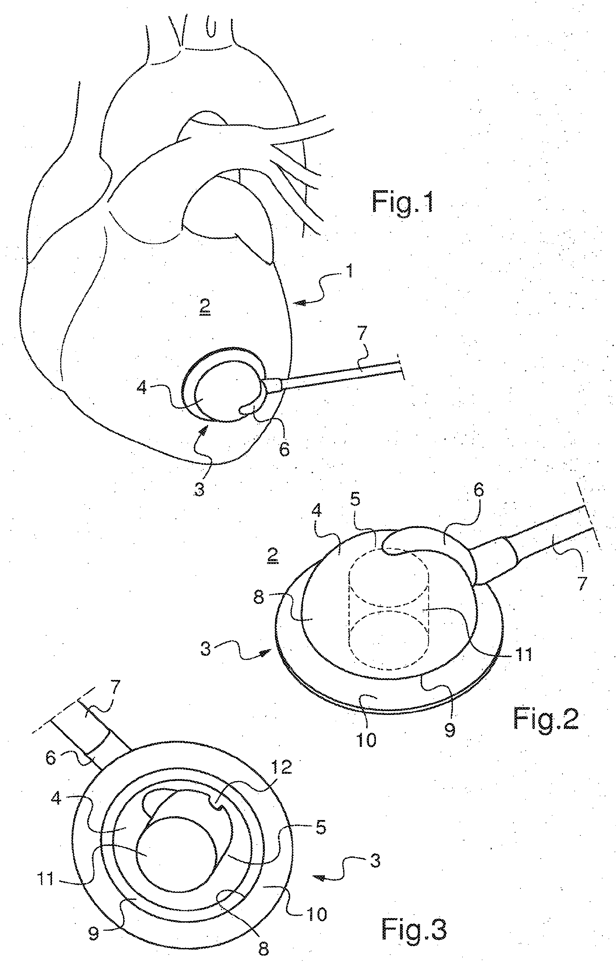

Sur la Figure 1, la référence 1 désigne un organe quelconque, en l'espèce un coeur supposé présenter une zone de tissu 2 siège d'une hémorragie importante, qu'il importe de contrôler et notamment de réduire sinon de stopper, pour éviter que le sang ne s'écoule en grande quantité à l'extérieur et/ou dans la cavité thoracique du patient, en pouvant mettre en cause le pronostic vital de l'individu, voire, plus immédiatement, gêner la vision et l'accès à une autre région de l'organe sur laquelle est pratiquée une intervention chirurgicale.

Bien entendu, le type d'organe traité n'importe pas en soi à l'invention qui s'applique de façon générale à toute lésion hémorragique, notamment pour les besoins d'une telle intervention ou en cas de blessure grave, occasionnant un écoulement sanguin à arrêter dans les meilleures conditions de rapidité et d'efficacité possibles.

Pour atteindre ce but, l'invention propose d'utiliser une ventouse perfectionnée 3, schématiquement représentée sur la Figure 1 dans la position qu'elle occupe lorsqu'elle est mise en place sur la zone du tissu 2 où se produit l'hémorragie, et dont les Figures 2 et 3 illustrent plus en détail les caractéristiques d'un premier mode de réalisation.

Comme on le voit sur ces Figures, la ventouse 3 considérée se présente sous la forme d'une coupelle sensiblement hémisphérique 4, de révolution, dont le fond 5, sensiblement au centre de sa partie supérieure, est relié de façon étanche, par un organe de raccordement 6 prolongé par un cathéter ou tube de liaison similaire 7, à un dispositif extérieur (non représenté) de mise sous vide du volume interne 8 de la coupelle, une fois celle-ci étroitement appliquée contre la zone de tissu 2 par son bord d'extrémité 9 opposé au fond 5, ce bord étant avantageusement prolongé par une collerette circulaire 10, plane et de préférence inclinée sur l'axe de la coupelle de manière à présenter un profil sensiblement conique, souple et apte à fléchir légèrement lorsque la ventouse 3 est mise en place.

Ce dispositif de mise sous vide du volume 8 par aspiration de l'air qu'il contient peut consister en une pompe à vide ou en un bocal d'aspiration sous vide du type « Redon », ce genre d'appareil, propre à fournir une dépression de l'ordre de 500 à 700 mm de Hg, étant en lui-même parfaitement classique dans la technique de sorte que sa description détaillée est ici inutile.

Selon l'invention et dans le premier mode de réalisation représenté, la coupelle 4 est associée, à l'intérieur du volume interne 8 qu'elle délimite, à un organe d'appui interne, constitué par un obturateur 11, plein et qui se présente sous la forme d'un cylindre droit dont l'axe est confondu avec celui de la coupelle, cet obturateur étant solidaire du fond 5, soit monobloc avec la coupelle, soit en étant réalisé séparément avant d'être collé ou soudé contre ce fond, au centre de celui-ci.

L'obturateur cylindrique 11 est réuni à l'organe de raccordement 6 et comporte au moins un orifice de communication 12 avec le volume interne 8 de la coupelle 4 pour le passage de l'air aspiré et évacué par le tube 7.

La coupelle 4 peut présenter une relative souplesse en étant dans ce cas réalisée en un matériau plastique du genre résine silicone ou en tout autre matière, biocompatible avec le tissu et apte à pouvoir être convenablement stérilisée avant d'être appliquée sur la zone 2. Elle peut aussi être réalisée avec un matériau rigide, tel qu'une résine epoxy durcie, de préférence avec un revêtement de silicone.

L'obturateur 11 est pour sa part réalisé en un matériau similaire à celui de la coupelle ou en un matériau différent, présentant une rigidité plus élevée. Cet obturateur présente généralement selon la direction de l'axe du cylindre qui le constitue, une hauteur sensiblement supérieure à celle de la coupelle 4, de manière à pouvoir exercer sur la zone de tissu en regard une compression convenable lorsque la ventouse 3 est mise en place, cette compression facilitant l'hémostase au sein de ce tissu et par suite le contrôle de l'hémorragie.

Toutefois, dans d'autres cas, où cette compression n'est pas souhaitable ou possible, la hauteur de l'obturateur 11 peut être inférieure à celle de la coupelle 4, mais néanmoins suffisante pour faciliter le positionnement de la ventouse 3 sur le tissu.

En règle ordinaire, la hauteur de la coupelle 4, selon les circonstances de son utilisation, est de l'ordre de 10 à 30 mm, la hauteur de l'obturateur pouvant selon le cas être plus ou moins augmentée ou diminuée de 2 à 5 mm. Le diamètre de la coupelle est compris entre 5 et 60 mm, en moyenne de l'ordre de 50 mm. L'obturateur 11 présente un diamètre extérieur qui est ordinairement voisin de la moitié de celui de la coupelle 4, au droit de son bord d'extrémité 9. La collerette circulaire souple 10, à profil conique et qui prolonge ce bord, présente une largeur de l'ordre de 3 à 6 mm environ.

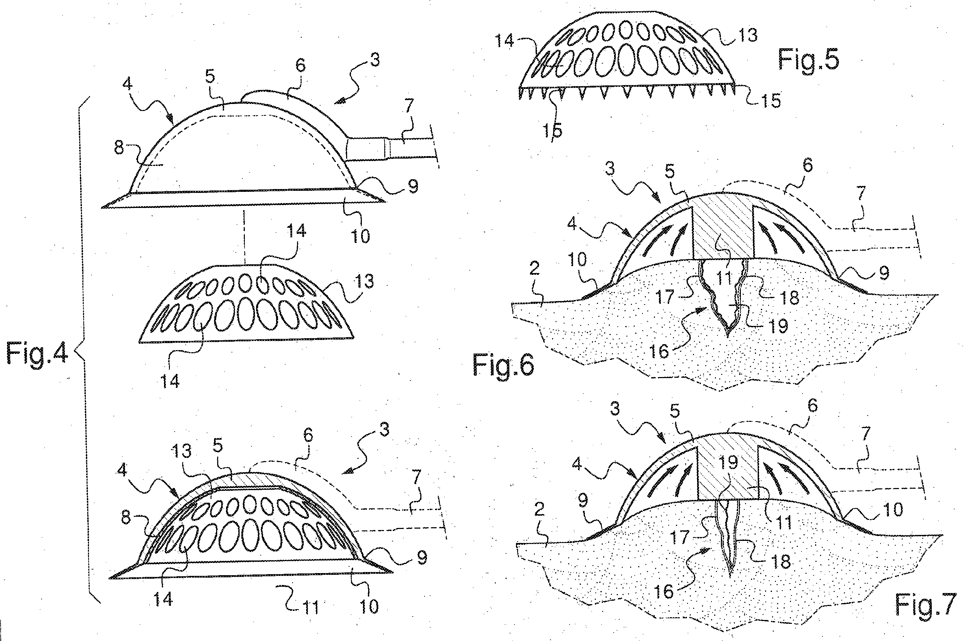

Dans une autre variante de réalisation de l'invention, l'organe d'appui interne de la ventouse 3 peut, comme illustré sur la vue éclatée de la Figure 4 qui permet de mieux voir ainsi la position relative des diverses pièces, consister en une grille 13, en forme de calotte sphérique, montée coaxialement dans la coupelle 4, cette grille, réalisée de préférence en un matériau plastique similaire à celui de la coupelle, présentant une pluralité de trous 14, notamment circulaires, régulièrement répartis dans sa surface, ces trous permettant le libre passage de l'air aspiré dans le volume interne 8 de la coupelle en évitant le bouchage éventuel du tube 7 par les déchets ou dépôts divers provenant de- la zone de tissu 2 recouverte par la ventouse 3. Dans d'autres réalisations pratiques, les trous 14 de la grille 13 pourraient présenter une forme différente et être répartis d'une autre façon dans la surface de cette grille.

Egalement, dans une autre variante illustrée sur la Figure 5, la ventouse 3 peut utiliser à nouveau une grille perforée similaire 13, mais dans laquelle la calotte qui forme cette grille comporte, dans son bord périphérique, des dents d'impactage en relief 15, aptes à s'ancrer superficiellement dans le tissu en regard pour mieux immobiliser la ventouse en position appropriée sur la zone de tissu à recouvrir et faciliter ensuite sa mise sous vide.

Les Figures 6 et 7 illustrent la manière dont la ventouse 3 de l'invention permet de réaliser l'hémostase rapide et efficace d'une zone de tissu 2, comportant, comme représenté sur la Figure 6, une plaie ouverte 16 dont les lèvres sensiblement en regard 17 et 18 forment entre elles une fente ou une ouverture schématiquement désignée sous la référence 19 et à travers laquelle se produit un écoulement sanguin à stopper dans les meilleures conditions de rapidité et d'efficacité.

La ventouse 3, comportant ici un obturateur central plein 11 selon la première variante décrite précédemment mais qui pourrait aussi bien être munie d'une grille interne perforée, équivalente, est appliquée sur la plaie 16, sa dimension diamétrale étant choisie de manière à ce qu'elle recouvre et entoure la fente ou ouverture 19 entre les lèvres 17 et 18, l'obturateur 11 porté par la ventouse à l'intérieur de celle-ci, étant centré et en contact avec la plaie, et selon sa dimension en hauteur, exerçant sur celle-ci une action plus ou moins importante, provoquant une légère compression sur ses lèvres.

Aussitôt mise en place, la ventouse 3 est reliée par son organe de liaison 6 et le cathéter 7 à son dispositif de mise sous vide extérieur, la combinaison de l'aspiration ainsi réalisée à l'intérieur de la ventouse (schématisée par les flèches apparaissant sur les Figures) et de l'appui du à l'obturateur interne 11, ayant pour effet de rapprocher les lèvres 17 et 18 l'une de l'autre en réduisant et finalement en annihilant la fente 19 comme illustré sur la Figure 7, ces lèvres se réunissant progressivement l'une à l'autre en assurant l'hémostase et la cicatrisation rapides et efficaces de la plaie 16, la dépression créée sous la ventouse 3 étant maintenue aussi longtemps que nécessaire.

On obtient ainsi une ventouse de conception très simple, d'un coût de fabrication réduit et surtout d'une très grande efficacité pour contrôler rapidement et de manière sûre une lésion hémorragique par réalisation d'une hémostase se maintenant dans le temps, facilitant dans la plus large mesure les interventions à effectuer sur le tissu à traiter, siège d'un écoulement sanguin.

Il va cependant de soi que l'invention ne se limite pas aux seuls exemples de réalisation décrits ci-dessus en référence aux dessins annexés ; elle en embrasse au contraire toutes les variantes entrant dans le champ des revendications qui suivent. Notamment, on pourrait prévoir de monter, sous une coupelle unique en forme de plaque souple, recouvrant une plus large zone de tissu, une pluralité d'obturateurs voisins, prévus en relief sur une seconde plaque, parallèle à la première et réunie de façon étanche à celle-ci à sa périphérie, des perçages étant ménagés dans la seconde plaque pour l'évacuation de l'air collecté entre les deux plaques dans un espace réuni à l'organe d'aspiration extérieur.

## Revendications

1. - Dispositif de contrôle hémostatique d'un écoulement sanguin, comportant une ventouse de recouvrement étanche, apte à entourer et à s'appliquer étroitement contre une zone de tissu (2) à traiter, siège d'une hémorragie ou d'un écoulement sanguin à contrôler, cette ventouse (3) venant en contact avec la périphérie de la zone de tissu et délimitant un volume interne (8) relié de façon étanche par un cathéter ou un tube de liaison similaire (7) à un organe extérieur d'aspiration et de mise sous vide, créant dans ce volume, entre la ventouse et la zone de tissu, une dépression de valeur déterminée, et dans lequel la ventouse (3) présente une forme générale de coupelle (4), dont le fond (5) supporte au moins un organe d'appui interne (11, 13), apte à venir se disposer en regard de la zone de tissu (2) sous la ventouse (3), **caractérisé en ce que** l'organe d'appui interne est constitué par un obturateur plein (11), solidarisé du fond (5) de la ventouse, la hauteur dudit obturateur plein (11) étant légèrement inférieure à celle de la coupelle (4) ou sensiblement supérieure à celle de la coupelle (4) à profil hémisphérique.

2. - Dispositif selon la revendication 1, **caractérisé en ce que** la coupelle (4) présente un profil hémisphérique autour d'un axe de révolution selon la direction duquel s'étend l'obturateur plein (11), disposé sensiblement au centre de la coupelle.

3. - Dispositif selon l'une des revendications 1 ou 2, **caractérisé en ce que** l'obturateur plein (11) est en forme de cylindre droit dont l'axe est confondu avec l'axe de révolution de la coupelle (4) .

4. - Dispositif selon l'une quelconque des revendications 1 à 3, **caractérisé en ce que** l'obturateur plein (11) comporte, dans le fond (5) de la coupelle (4), un organe de raccordement étanche (6) avec le tube de liaison (7) relié à l'organe extérieur de mise sous vide et au moins un orifice de communication (12) avec le volume interne (8) de la coupelle (4) pour l'aspiration de l'air contenu dans ce volume.

5. - Dispositif selon l'une quelconque des revendications 1 à 4, **caractérisé en ce que** la coupelle (4) et l'obturateur plein (11) forment un ensemble monobloc.

6. - Dispositif selon l'une quelconque des revendications 1 à 4, **caractérisé en ce que** la coupelle (4) et l'obturateur plein (11) sont réalisés séparément puis collés mutuellement dans le fond de la coupelle.

7. - Dispositif selon l'une quelconque des revendications 1 à 6, **caractérisé en ce que** le diamètre de l'obturateur plein (11) est sensiblement égal à la moitié du diamètre de la coupelle (4) au droit de son bord (9) appliqué contre la zone de tissu (2).

8. - Dispositif de contrôle hémostatique d'un écoulement sanguin, comportant une ventouse de recouvrement étanche, apte à entourer et à s'appliquer étroitement contre une zone de tissu (2) à traiter, siège d'une hémorragie ou d'un écoulement sanguin à contrôler, cette ventouse (3) venant en contact avec la périphérie de la zone de tissu et délimitant un volume interne (8) relié de façon étanche par un cathéter ou un tube de liaison similaire (7) à un organe extérieur d'aspiration et de mise sous vide, créant dans ce volume, entre la ventouse et la zone de tissu, une dépression de valeur déterminée, et dans lequel la ventouse (3) présente une forme générale de coupelle (4) avec un organe d'appui interne, apte à venir se disposer en regard de la zone de tissu (2) sous la ventouse (3), ledit organe d'appui interne étant constitué par une grille perforée (13) présentant une pluralité de trous de passage (14) permettant la libre traversée de l'air dans le volume interne (8) de la coupelle avant son évacuation dans le tube de liaison (7) par l'organe extérieur de mise sous vide, **caractérisé en ce que** le fond (5) de ladite coupelle (4) supporte au moins ledit organe d'appui interne (11, 13).

9. - Dispositif selon la revendication 8, **caractérisé en ce que** les trous de passage (14) de la grille (13) sont identiques ou non, circulaires ou présentent un autre profil et le cas échéant sont régulièrement répartis dans son étendue.

10. - Dispositif selon l'une des revendications 8 ou 9, caractérisé en ce la périphérie de la grille (13) comporte des dents d'impactage (15) en relief, de faible hauteur, propres à pénétrer légèrement dans la couche superficielle de la zone de tissu (2), lors de l'application de la coupelle (4) sur celle-ci.

11. - Dispositif selon l'une quelconque des revendications 1 à 10, **caractérisé en ce que** le bord (9) de la coupelle (4) opposé à son fond (5), comporte une collerette d'appui plane (10), circulaire, en contact la zone de tissu (2) qu'elle entoure.

12. - Dispositif selon la revendication 11, **caractérisé en ce que** la collerette d'appui plane (10) présente un profil sensiblement conique sur l'axe de la coupelle.

13. - Dispositif selon l'une quelconque des revendications 1 à 12, caractérisé en ce la coupelle (4) est souple et réalisée en un matériau du genre résine silicone ou analogue, biocompatible avec la zone de tissu à traiter.

14. - Dispositif selon l'une quelconque des revendications 1 à 12, caractérisé en ce la coupelle (4) est constituée au moyen d'un matériau rigide, du type résine epoxy durcie, avec revêtement de protection adhésif en silicone.

15. - Dispositif selon l'une quelconque des revendications 1 à 14, caractérisé en ce le diamètre de la coupelle (4) au droit de son bord (9) appliqué contre la zone de tissu (2) est compris entre 5 et 60 mm, de préférence et en moyenne de l'ordre de 50mm.

16. - Dispositif selon l'une quelconque des revendications 1 à 15, **caractérisé en ce que** la hauteur de la coupelle (4) est comprise entre 10 et 30mm.

17. - Dispositif selon l'une quelconque des revendications 1 à 16, **caractérisé en ce que** l'organe extérieur de mise sous vide est constitué par une pompe ou un bocal d'aspiration du type « Redon ».

18. - Dispositif selon la revendication 17, **caractérisé en ce que** la dépression créée par l'organe extérieur est comprise entre 500 et 700 mm de Hg.

## Patentansprüche

1. Vorrichtung zur hämostatischen Blutflusskontrolle, umfassend ein dicht abdeckendes Saugventil, das geeignet ist, eine zu behandelnde Gewebezone (2), an der sich eine Hämorrhagie oder ein Blutfluss, die kontrolliert werden müssen, befindet, zu umgeben und sich eng an diese anzulegen, wobei dieses Saugventil (3) mit der Peripherie der Gewebezone in Kontakt kommt und ein Innenvolumen (8) begrenzt, das dicht durch einen Katheter oder eine ähnliche Verbindungsröhre (7) mit einem äußeren Ansaug- und Vakuumelement verbunden ist, das in diesem Volumen zwischen dem Saugventil und der Gewebezone einen Unterdruck mit bestimmtem Wert erzeugt, und wobei das Saugventil (3) eine allgemeine Kuppelform (4) aufweist, deren Boden (5) mindestens ein inneres Stützelement (11, 13) trägt, das geeignet ist, sich gegenüber der Gewebezone (2) unter dem Saugventil (3) anzuordnen, **dadurch gekennzeichnet, dass** das innere Stützelement von einem vollen Verschlusselement (11) gebildet ist, das mit dem Boden (5) des Saugventils verbunden ist, wobei die Höhe des vollen Verschlusselements (11) etwas geringer als jene der Kuppel (4) oder im Wesentlichen größer als jene der Kuppel (4) mit halbkugelförmigem Profil ist.

2. Vorrichtung nach Anspruch 1, **dadurch gekennzeichnet, dass** die Kuppel (4) ein halbkugelförmiges Profil um eine Umdrehungsachse aufweist, entlang der sich das volle Verschlusselement (11) erstreckt, das im Wesentlichen in der Mitte der Kuppel angeordnet ist.

3. Vorrichtung nach einem der Ansprüche 1 oder 2, **dadurch gekennzeichnet, dass** das volle Verschlusselement (11) die Form eines geraden Zylinders hat, dessen Achse mit der Umdrehungsachse der Kuppel (4) zusammenfällt.

4. Vorrichtung nach einem der Ansprüche 1 bis 3, **dadurch gekennzeichnet, dass** das volle Verschlusselement (11) im Boden (5) der Kuppel (4) ein Element (6) für den dichten Anschluss an das Verbindungsrohr (7), das mit dem äußeren Vakuumelement verbunden ist, und mindestens eine Öffnung (12) zur Verbindung mit dem Innenvolumen (8) der Kuppel (4) für die Ansaugung der in diesem Volumen enthaltenen Luft umfasst.

5. Vorrichtung nach einem der Ansprüche 1 bis 4, **dadurch gekennzeichnet, dass** die Kuppel (4) und das volle Verschlusselement (11) eine einstückige Einheit bilden.

6. Vorrichtung nach einem der Ansprüche 1 bis 4, **dadurch gekennzeichnet, dass** die Kuppel (4) und das volle Verschlusselement (11) getrennt hergestellt und dann im Boden der Kuppel zusammengeklebt werden.

7. Vorrichtung nach einem der Ansprüche 1 bis 6, **dadurch gekennzeichnet, dass** der Durchmesser des vollen Verschlusselements (11) im Wesentlichen gleich der Hälfte des Durchmessers der Kuppel (4) im rechten Winkel zu ihrem Rand (9), der an die Gewebezone (2) angelegt wird, ist.

8. Vorrichtung zur hämostatischen Blutflusskontrolle, umfassend ein dicht abdeckendes Saugventil, das geeignet ist, eine zu behandelnde Gewebezone (2), an der sich eine Hämorrhagie oder ein Blutfluss, die kontrolliert werden müssen, befindet, zu umgeben und sich eng an diese anzulegen, wobei dieses Saugventil (3) mit der Peripherie der Gewebezone in Kontakt kommt und ein Innenvolumen (8) begrenzt, das dicht durch einen Katheter oder eine ähnliche Verbindungsröhre (7) mit einem äußeren Ansaug- und Vakuumelement verbunden ist, das in diesem Volumen zwischen dem Saugventil und der Gewebezone einen Unterdruck mit bestimmtem Wert erzeugt, und bei der das Saugventil (3) eine allgemeine Kuppelform (4) mit einem inneren Stützelement aufweist, das geeignet ist, sich gegenüber der Gewebezone (2) unter dem Saugventil (3) anzuordnen, wobei das innere Stützelement von einem perforierten Gitter (13) gebildet ist, das eine Vielzahl von Durchgangslöchern (14) aufweist, die das freie Strömen der Luft durch das Innenvolumen (8) der Kuppel vor ihrer Ableitung in die Verbindungsröhre (7) durch das äußere Vakuumelement ermöglicht, **dadurch gekennzeichnet, dass** der Boden (5) der Kuppel (4) mindestens das innere Stützelement (11, 13) trägt.

9. Vorrichtung nach Anspruch 8, **dadurch gekennzeichnet, dass** die Durchgangslöcher (14) des Gitters (13) identisch sind oder nicht, kreisförmig sind oder ein anderes Profil aufweisen und gegebenenfalls regelmäßig auf seiner Fläche verteilt sind.

10. Vorrichtung nach einem der Ansprüche 8 oder 9, **dadurch gekennzeichnet, dass** die Peripherie des Gitters (13) reliefartige Einschlagzähne (15) von geringer Höhe umfasst, die geeignet sind, leicht in die oberflächliche Schicht der Gewebezone (2) einzudringen, wenn die Kuppel (4) an diese angelegt wird.

11. Vorrichtung nach einem der Ansprüche 1 bis 10, **dadurch gekennzeichnet, dass** der Rand (9) der Kuppel (4), der ihrem Boden (5) gegenüberliegt, einen flachen kreisförmigen Stützkragen (10) umfasst, der mit der Gewebezone (2), die sie umgibt, in Kontakt ist.

12. Vorrichtung nach Anspruch 11, **dadurch gekennzeichnet, dass** der flache Stützkragen (10) ein im Wesentlichen konisches Profil auf der Achse der Kuppel aufweist.

13. Vorrichtung nach einem der Ansprüche 1 bis 12, **dadurch gekennzeichnet, dass** die Kuppel (4) biegsam und aus einem Material von der Art Silikonharz oder dergleichen, das mit der zu behandelnden Gewebezone biologisch vereinbar ist, herstellt ist.

14. Vorrichtung nach einem der Ansprüche 1 bis 12, **dadurch gekennzeichnet, dass** die Kuppel (4) mit Hilfe eines starren Materials vom Typ gehärtetes Epoxidharz mit einer Haftschutzverkleidung aus Silikon ausgeführt ist.

15. Vorrichtung nach einem der Ansprüche 1 bis 14, **dadurch gekennzeichnet, dass** der Durchmesser der Kuppel (4) im rechten Winkel zu ihrem Rand (9), der an der Gewebezone (2) anliegt, zwischen 5 und 60 mm, vorzugsweise und durchschnittlich ungefähr 50 mm beträgt.

16. Vorrichtung nach einem der Ansprüche 1 bis 15, **dadurch gekennzeichnet, dass** die Höhe der Kuppel (4) zwischen 10 und 30 mm beträgt.

17. Vorrichtung nach einem der Ansprüche 1 bis 16, **dadurch gekennzeichnet, dass** das äußere Vakuumelement von einer Ansaugpumpe oder einem Ansauggefäß vom Typ "Redon" gebildet ist.

18. Vorrichtung nach Anspruch 17, **dadurch gekennzeichnet, dass** der durch das äußere Element erzeugte Unterdruck zwischen 500 und 700 mm Hg beträgt.

## Claims

1. A device for the haemostatic control of bleeding, including a tight collecting cup, which is able to surround and be tightly applied onto a tissue area (2) to be treated, where a haemorrhage of a bleeding episode to be controlled takes place, wherein this suction cup (3) comes into contact with the periphery of the tissue area and defines an inner volume (8) which is tightly linked through a catheter or similar linking tube (7) to an outer suction and vacuum creating device, thus creating in this volume, between the suction cup and the tissue area, a depression having a defined value, and wherein the suction cup (3) has a general shape of a dome (4), the bottom of which (5) supports at least one inner supporting device (11,13), which is able to be placed opposing the tissue area (2) under the suction cup (3), **characterized in that** the inner supporting device is made up by a solid obturator (11), linked to the bottom (5) of the suction cup, the height of said solid obturator (11) being slightly below that of the dome (4) or significantly higher than that of the dome (4) which has a hemispheric profile.

2. A device according to claim 1, **characterized in that** the dome (4) has a hemispheric profile around a rotating axis in the direction of which the solid obturator (11) is placed, located essentially at the center of the dome.

3. A device according to any one of claims 1 or 2, **characterized in that** the solid obturator (11) is in the shape of a straight cylinder the axis of which is the same as the rotating axis of the dome (4).

4. A device according to any one of claims 1 to 3, **characterized in that** the solid obturator (11) includes, at the bottom(5) of the dome (4), a water-tight linking device (6) for connection with the linking tube (7) which is linked to the outer device for creating vacuum, and with at least one opening (12) for communicating with the inner volume (8) of the dome (4) for drawing up the air which is contained in this volume.

5. A device according to any one of claims 1 to 4, **characterized in that** the dome (4) and the solid obturator (11) form a single-piece unit.

6. A device according to any one of claims 1 to 4, **characterized in that** the dome (4) and the solid obturator (11) are manufactured separately, then glued together at the bottom of the dome.

7. A device according to any one of claims 1 to 6, **characterized in that** the diameter of the solid obturator (11) is significantly equal to the half of the dome diameter (4) directly above its edge (9) which is applied onto the tissue area (2).

8. A device for the haemostatic control of bleeding, including a tight collecting cup, which is able to surround and be tightly applied onto a tissue area (2) to be treated, where a haemorrhage of a bleeding episode to be controlled takes place, wherein this suction cup (3) comes into contact with the periphery of the tissue area and defines an inner volume (8) which is tightly linked through a catheter or similar linking tube (7) to an outer suction and vacuum creating device, thus creating in this volume, between the suction cup and the tissue area, a depression having a defined value, and wherein the suction cup (3) has a general shape of a dome (4) with an inner supporting device, which is able to be placed opposing the tissue area (2) under the suction cup (3), said inner supporting device is made up by a perforated grid (13) with a plurality of through holes (14) freely letting the air into the inner volume (8) of the dome before it is discharged into the linking tube (7) through the outer device for creating a vacuum, **characterized in that** the bottom of said dome (4) supports at least said inner supporting device (11,13).

9. A device according to claim 8, **characterized in that** the through holes (14) of the grid (13) are or not identical, circular or present another profile, and if need be are regularly distributed within its surface.

10. A device according to any one of claims 8 or 9, **characterized in that** the periphery of the grid (13) includes projecting embossed contact teeth (15) of low height, which are able to slightly penetrate into the superficial layer of the tissue area (2) when the dome (4) is applied onto said area.

11. A device according to any one of claims 1 to 10, **characterized in that** the edge (9) of the dome (4) as opposed to its bottom (5), includes a plane circular supporting flange (10) in contact with the tissue area (2) which it surrounds.

12. A device according to claim 11, **characterized in that** the plan supporting flange (10) has a profile which is significantly conical on the axis of the dome.

13. A device according to any one of claims 1 to 12, **characterized in that** the dome (4) is flexible and manufactured in a silicone resin-like material or similar, and is biocompatible with the tissue area to be treated.

14. A device according to one of claims 1 to 12, **characterized in that** the dome (4) is manufactured from a rigid material such as a hardened epoxy resin, with an adhesive silicone protection coating.

15. A device according to any one of claims 1 to 14, **characterized in that** the diameter of the dome (4) directly above its edge (9) as applied onto the tissue area (2) is between 5 and 60 mm, preferably and on average about 50 mm.

16. A device according to any one of claims 1 to 15, **characterized in that** the height of the dome (4) is between 10 and 30 mm.

17. A device according to any one of claims 1 to 16, **characterized in that** the outer vacuum-creating device is made up by a vacuum pomp or a "Redon" type suction bowl.

18. A device according to claim 17, **characterized in that** the depression as created by the outer device is comprised between 500 and 700 mm of Hg.
